(19) Europäisches Patentamt
European Patent Office
Office européen des brevets

(11) EP 0 486 046 B1

(12) **EUROPÄISCHE PATENTSCHRIFT**

(45) Veröffentlichungstag und Bekanntmachung des Hinweises auf die Patenterteilung:
**01.05.1996 Patentblatt 1996/18**

(51) Int. Cl.$^6$: **C07C 57/30**, A61K 31/205, C07C 215/10, C07H 5/06

(21) Anmeldenummer: **91119523.8**

(22) Anmeldetag: **15.11.1991**

(54) **Komplexe, enthaltend S(+)-Phenyl-alkansäuren und Aminozucker**

S(+)-Phenyl-alkanoic acids and aminosugar containing complexes

Complexes contenant acides S(+)-phénylalkanoiques et l'amino-sucre

(84) Benannte Vertragsstaaten:
**AT BE CH DE DK FR GB IT LI LU NL SE**

(30) Priorität: **15.11.1990 DE 4036460**

(43) Veröffentlichungstag der Anmeldung:
**20.05.1992 Patentblatt 1992/21**

(73) Patentinhaber: **MEDICE Chem.-Pharm. Fabrik Pütter GmbH & Co. KG**
**D-58638 Iserlohn (DE)**

(72) Erfinder: **Paradies, Henrich Hasko, Prof. Dr. Dr.**
**W-5860 Iserlohn (DE)**

(74) Vertreter: **Reinhard - Skuhra - Weise & Partner**
**Postfach 44 01 51**
**D-80750 München (DE)**

(56) Entgegenhaltungen:
**EP-A- 0 398 288**
**DE-A- 2 103 387**
**DE-A- 3 205 077**
**DE-A- 3 639 038**
**DE-A- 3 700 172**

- **CHEMICAL ABSTRACTS, Band 102, Nr. 26, 1985, Seite 336, Spalte 1, Zusammenfassung Nr. 225919f, Columbus, Ohio, US; A. FINI et al.: "Dissolution profiles of NSAID carboxylic acids and their salts with different counter ions", & PHARM. ACTA HELV. 1985, 60(2), 58-62**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 486 046 B1

Printed by Rank Xerox (UK) Business Services
2.11.3/3.4

## Beschreibung

Die vorliegende Erfindung betrifft wasserstoffbrücken-gebundene Komplexe mit einer Stöchiometrie von 1:1 aus S-(+)-Phenyl-alkansäuren und Aminozuckern.

Zum Stande der Technik wird verwiesen auf CA, 1985, 102, 225 919 und die DE-OS 21 03 387.

Zu dieser DE-OS 21 03 387 wird weiter unten Stellung genommen.

Eine Aufgabe der vorliegenden Erfindung ist es, neue Stoffe auf der Basis von S-(+)-Phenyl-alkansäuren und Aminozuckern zu schaffen und deren vorteilhafte Verwendung in pharmazeutischen Zubereitungen zu erarbeiten.

Diese Aufgabe wird erfindungsgemäß gelöst durch wasserstoffbrücken-gebundene Komplexe mit einer Stöchiometrie von 1:1 aus S-(+)-Phenyl-alkansäuren und Aminozuckern, bei welchen die Komplex-Bindung auf Wechselwirkungen zwischen der Karboxylgruppe der S-(+)-Phenyl-alkansäuren und der Hydroxylgruppe am Kohlenstoffatom ($C_3$) der Aminozucker mit einem Proton-Switch der Form

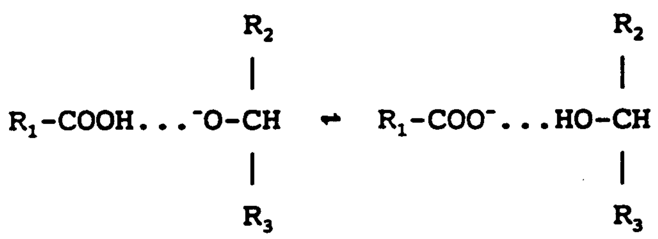

beruht, wobei $R_1$-COOH die S-(+)-Phenyl-alkansäuren und

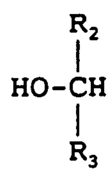

die Aminozucker bedeuten, und wobei die pKa-Werte bezüglich der Karboxylgruppe der S-(+)-Phenyl-alkansäuren im Bereich von 3,5 - 3,9 und die pKa-Werte bezüglich der Hydroxylgruppe am Kohlenstoffatom ($C_3$) der Aminozucker im Bereich von 1,9 - 4,0 liegen.

Die DE-A-32 05 077 beschreibt Salze von Phenylalkansäuren mit beispielsweise Meglumin. Die erfindungsgemäßen Komplexe werden nicht offenbart.

Die EP 0 398 288 mit einer US-Priorität vom 16.5.1989, veröffentlicht am 22.11.1990, gehört zum Stand der Technik nach Art. 54(3) (4) EPÜ. Die Anmeldung beschreibt zwar Komplexe von S-(+)-Phenylalkansäuren mit Aminozuckern. Die erfindungsgemäß wichtigen pKa-Werte der Karboxylgruppe der S-(+)-Phenylalkansäuren und der Hydroxylgruppe am Kohlenstoffatom 3 der Aminozucker werden jedoch nicht offenbart. Die vorliegende Anmeldung ist somit die erste Anmeldung im Sinne von Art. 87(1) EPÜ. Aufgrund der vorgenannten Unterschiede ist der Gegenstand der Anmeldung gegenüber der EP-A-0 398 288 auch neu.

Die DE-A-2 103 387 offenbart einfach Kombinationen von Glucosaminhydrochlorid mit einem oder mehreren Antirheumatika. Die erfindungsgemäß beanspruchten Komplexe werden nicht genannt.

Pharm. Acta Helv. 60, Nr. 2 (1985), S. 58-62 beschreibt Salze von Phenylalkansäuren mit Aminozuckern. Die erfindungsgemäßen Komplexe sind nicht beschrieben.

Die DE-A-37 00 172 beschreibt Salze von Aminozuckern mit Phenylalkansäuren. Die erfindungsgemäßen Komplexe werden nicht offenbart.

Die DE-A-36 39 038 beschreibt die bevorzugte Verwendung von S-(+)-Ibuprofen. Die Kombination mit einem Aminozucker oder eine Komplexbildung wird nicht beschrieben.

Vorzugsweise sollen hier als S-(+)-Phenyl-alkansäuren auch S-(+)-Ibuprofen oder S-(+)-Naproxen verstanden und verwendet werden.

Vorzugsweise sollen hier als S-(+)-Phenyl-alkansäuren auch die nachfolgend beschriebenen Substanzen verstanden und verwendet werden. Diesen Verbindungen ist die untenstehende Strukturformel gemeinsam,

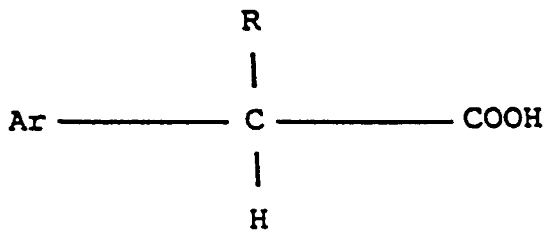

in welcher R ein Alkylrest ist, Ar vorzugsweise eine monozyklische, polyzyklische oder ortho-kondensierte polyzyklische aromatische Gruppe mit bis zu 12 Kohlenstoffatomen in dem aromatischen System, z.B. Phenyl, Diphenyl und Naphtyl. Die Substituenten dieser aromatischen Gruppen enthalten ein oder mehrere Halogenatome, $C_1$-$C_4$-Alkyle, Benzyl, Hydroxy, $C_1$-$C_2$-Alkoxy, Phenoxy und Benzoyl-Gruppen. Beispiele dieser substituierten Aryle sind: 4-Isobutyl-phenyl, 3-Phenoxy-phenyl, 2-Fluoro-4-diphenyl, 4'-Fluoro-4-diphenyl, 6-Methoxy-2-naphthyl, 5-Chloro-6-methoxy-2-naphthyl und 5-Bromo-6-methoxy-naphthyl, 4-Chloro-phenyl, 4-Difluoro-methoxy-phenyl, 6-Hydroxy-2-naphthyl und 5-Bromo-6-hydroxy-2-naphthyl.

Vorzugsweise weisen die Aminozucker die folgende allgemeine Formel

Z-NHR,

auf, wobei

R = Wasserstoff, Methyl oder Ethyl und
Z = das Gerüst des Aminozuckers, der 5 oder 6 Kohlenstoffatome enthält.

Vorzugsweise ist der Aminozucker linear oder zyklisch.

Vorzugsweise ist der Aminozucker ein Pentose- oder Hexosederivat, insbesondere Glukamin, N-Methyl-Glukamin, N-Ethyl-Glukamin, Ribamin, vorzugsweise in der D-Form, sowie die Epimeren der Hexosamine, insbesondere Allosamin, Altrosamin, Glukosamin, Mannosamin, Gulosamin, Idosamin, Galaktosamin und Talosamin und der Pentosamine, insbesondere Ribosamin, Arabinosamin, Xylosamin und Lyxosamin.

Vorzugsweise liegen die Aminozucker in der D-Form vor.

Erfindungsgemäß werden die erfindungsgemäßen Komplexe durch folgende Verfahrensschritte hergestellt:

a) zur Herstellung aus wäßrigem Medium (nur Wasser) oder schwach gepufferten wäßrigen Lösungen, die einen pH-Bereich umfassen zwischen pH 5.5-7.5 (20°C) bereitet man eine gepufferte, wäßrige Lösung, z.B. einen 0.01M-0.001 M-$K_2HPO_4$/$KH_2PO_4$-Puffer pH 6.0-7.5 (20°C) vor, in die unter stetem Rühren eine äquivalente Menge S-(+)-Phenyl-alkansäure eingetragen wird;
b) die Lösung wird unter stetem Rühren auf 40°C (Wasserbad) erwärmt, bis eine klare, transparente Lösung erhalten wird (normalerweise nach 20 Minuten) und alle S-(+)-Phenyl-alkansäure in Lösung gegangen ist;
c) anschließend wird der pH der Lösung auf pH 5.5-6.O unter Zugabe von verdünnter Phosphorsäure ($H_3PO_4$) eingestellt (20°C) und danach wird die äquivalente (entsprechende) Menge der Aminozucker unter stetem Rühren eingetragen;
d) die Komplexbildung ist nach 20 Minuten beendet, worauf nach Abkühlen auf 0-4°C die Komplexe kristallin anfallen und über eine Glasnutsche oder einen Glasfilter (1G4) von der Mutterlauge abgetrennt werden können;
e) alternativ zu Verfahrensschritt d) kann man die klare Lösung im Rotationsverdampfer (Wasserbad-Temperatur 25 - 30°C) im Wasserstrahl-Vakuum auf die Hälfte des Volumens eingeengt werden, worauf sich ein farbloser (amorpher) Niederschlag bildet, der über einen 1G4-Glasfilter abfiltriert aus Wasser/Ethanol ($^{70}/_{30}$ $^{V}/_{V}$) oder aus Ethylacetat (100 %) umkristallisiert werden kann.

Bei den erfindungsgemäßen Stoffen handelt es sich nicht um eine Salzbildung zwischen einer sauren Gruppierung (Karboxyl-Gruppe des Ibuprofens) und einem basischen Rest der Aminozucker, sondern, wie die Röntgenstrukturanalyse und die FT-IR-Spektren zeigen, um Karboxylat-Karboxyl-Wechselwirkungen, wobei sich beide Karboxyl-Reste des Aminozuckers und z.B. des Ibuprofens, ein Proton teilen. D.h. der Komplex wird entsprechend der Röntgenstrukturanalyse durch eine Wasserstoffbrücke gebildet.

Die erfindungsgemäßen Komplexe können vorteilhaft in pharmazeutischen Zubereitungen verwendet werden, enthaltend einen oder mehrere Komplexe und gegebenenfalls wahlweise zusätzlich physiologisch verträgliche gebräuchliche Streckungsmittel oder Träger.

Insbesondere vorteilhaft ist eine pharmazeutische Zubereitung auf der Grundlage von Phenyl-alkansäuren mit antiinflammatorischer, antipyretischer, antimikrobieller und analgetischer Wirkung, enthaltend einen Wirkstoffkomplex aus einer Phenyl-alkansäure und einem Aminozucker und ggf. zusätzlich übliche und physiologisch verträgliche Hilfsstoffe, bei der der Wirkstoffkomplex aus S-(+)-Phenyl-alkansäuren und Aminozuckern besteht.

Insbesondere vorteilhaft ist eine pharmazeutische Zubereitung auf der Grundlage von Ibuprofen oder Naproxen mit antiinflammatorischer, antipyretischer, antimikrobieller und analgetischer Wirkung, enthaltend einen Wirkstoffkomplex aus einer Ibuprofen oder Naproxen und Aminozuckern und ggf. zusätzlich übliche und physiologisch verträgliche Hilfsstoffe, bei der der Wirkstoffkomplex aus S-(+)-Ibuprofen oder S-(+)-Naproxen und einem Aminozucker besteht und einen Gewichtsanteil 0,1 bis 90% (w/w) der Zusammensetzung ausmacht.

Insbesondere vorteilhaft ist eine pharmazeutische Zusammensetzung, die 50 bis 800 mg, vorzugsweise 100 bis 600 mg, insbesondere 100 bis 300 mg S-(+)-Ibuprofen bzw. S-(+)-Naproxen enthält.

Insbesondere vorteilhaft ist eine pharmazeutische Zubereitung, bei der die geeignete Dosis für die orale oder die parenterale Verabreichung im Bereich von 50 - 1200 mg pro Tag, normalerweise zwischen 100 und 800 mg pro Tag, vorzugsweise zwischen 200 und 600 mg S-(+)-Ibuprofen pro Tag liegt und bei der die geeignete Dosis bei einer topischen Anwendung des Komplexes im Bereich von 10 - 200 mg pro Tag liegt.

Im folgenden wird die "pharmazeutisch aktive Verbindung" im weiteren Sinne als ein Komplex bezeichnet. In der medizinischen Anwendung kann diese pharmazeutisch aktive Verbindung oral, rektal, parentaral oder topisch angewendet werden, insbesondere jedoch oral oder topisch. Daher kann die therapeutische Zusammensetzung der vorliegenden Erfindung jede an sich bekannte pharmazeutische Zubereitung für orale, rektale, parenterale oder topische Anwendungen sein. Pharmazeutische gebräuchliche Träger, welche in solchen pharmazeutischen Zusammensetzungen angewendet werden, sind in der Pharmazie häufig beschrieben. Die Zusammensetzung dieser Erfindung kann 0,1 - 90% (w/w) der aktiven Verbindung entsprechen. Den Zusammensetzungen entsprechen normale, einheitliche Dosierungsformen. Diese Dosierungsformen enthalten 50 - 800 mg, vorzugsweise 100 - 600 mg oder 100 - 300 mg S-(+)-Ibuprofen.

Es werden orale Verabreichungsformen gemäß dieser Erfindung bevorzugt, wie z. B. Tabletten, Kapseln, Sirup und wäßrige oder ölige Suspensionen. Tabletten können z. B. hergestellt werden durch Mischen der aktiven Verbindung mit inerten Streckungsmitteln wie z. B. Calciumphosphat in Gegenwart eines aufschließenden Hilfsstoffes z. B. Stärke, oder Schmiermittels, z. B. Magnesiumstearat mit anschließender Tablettierung im normalen Herstellungssinne. Die Tabletten können in Form einer Retardformulierung der aktiven Verbindung nach bekannten Methoden hergestellt werden. Falls wünschenswert, können solche Tabletten nach entsprechend bekannten Methoden hergestellt werden, die sich im Magen nicht zersetzen; zum Beispiel unter Zuhilfenahme von Cellulose, Acetat, Phthalat. Entsprechend können Kapseln, z. B. Weich- und Hartgelatine-Kapseln hergestellt werden, welche die pharmazeutisch aktive Verbindung allein oder in Gegenwart von zugegebenen Hilfsstoffen enthalten. Diese Kapseln können mit üblicher pharmazeutischer Technologie, mit oder ohne magenresistenter Umhüllung, hergestellt werden. Andere Zusammensetzungen für orale Verabreichung schließen wäßrige Lösungen, die die aktive pharmazeutische Verbindung enthalten, in Gegenwart eines nicht toxischen Suspendierungsmittels, z. B. Carboxymethylcellulose und ölige Suspensionen, welche die aktive pharmazeutische Verbindung in Gegenwart eines Pflanzenöls.

Für die topische Anwendung der aktiven pharmazeutischen Verbindung können entsprechend dieser Erfindung pharmazeutische Formulierungen eingesetzt werden. Die pharmazeutisch aktive Verbindung wird in diesem Falle in einer pharmazeutisch geeigneten Creme, Salbe oder einem Gel dispergiert. Eine geeignete Creme kann z. B. dadurch hergestellt werden, daß die aktiv pharmazeutische Verbindung in einem topischen Träger wie z. B. leichtflüssiges Paraffin in einem wäßrigen Medium unter Zuhilfenahme von oberflächenaktiven Stoffen (Detergentien) dispergiert werden. Eine Salbe kann z. B. durch Mischen der pharmazeutisch aktiven Verbindung mit einem topischen Träger, wie z. B. Mineralöl oder Paraffin oder Bienenwachs hergestellt werden. Eine gelartige Formulierung kann durch Mischen einer aktiven pharmazeutischen Verbindung mit einem topischen Träger, z. B. Carbomer BP, in Gegenwart von Wasser hergestellt werden. Topisch anwendbare Zusammensetzungen können u.a. aus einer Matrix bestehen, die in der Lage ist, die aktive pharmazeutische Verbindung so zu dispergieren, daß diese durch ihren engen Kontakt mit der Haut transdermal verabreicht wird. Eine geeignete transdermale Zusammensetzung kann u. a. durch Mischen der pharmazeutisch aktiven Verbindung mit einem topischen Träger, wie vorne beschrieben, zusammen mit einem möglichen transdermalen Beschleuniger, z. B. Dimethylsulfoxid oder Propylenglykol, hergestellt werden.

Pharmazeutische Formulierungen entsprechend dieser Erfindung, die geeignet sind für die rektale Anwendung sind u. a. Suppositorien auf der Basis von Polyethylenglykol oder Kakaobutter.

Pharmazeutische Formulierungen zur parenteralen Anwendung beinhalten bekannte pharmazeutische Formulierungen z. B. sterile Suspensionen oder sterile Lösungen in einem geeigneten Lösungsmittel.

In einigen speziellen pharmazeutischen Formulierungen scheint es sinnvoll zu sein, die pharmazeutischen aktiven Verbindungen in einer Größe von kleinen Partikeln zu haben. z. B. kolloidale Lösungen oder partikuläre Suspensionen in einer Größenordnung von 0,1 - 1 µm (Kolloidmühle).

Wenn wünschenswert, können entsprechend dieser Erfindung auch Zusammensetzungen mit anderen kompatiblen pharmazeutischen Wirkstoffen hergestellt werden.

Diese Komplexe entsprechend der Erfindung haben antiinflammatorische, antipyretische und interessante antimikrobielle Eigenschaften sowie analgetische Effekte. Diese Komplexe haben u. a. den Vorteil, daß sie nach oraler Verabreichung nach kürzester Zeit wesentlich höhere Plasmaspiegel von S-(+)-Ibuprofen liefern als S-(+)-Ibuprofen in Form der freien Säure. Daher sind diese Komplexe in der Anwendung besonders wichtig zur Behandlung des akuten Schmerzes, wobei eine schnelle Anflutung mit sofortiger Schmerzbefreiung erzielt werden kann. Die Behandlung von Entzündungen und Schmerzen ist insbesondere wichtig bei Rheumapatienten, mit Indikationen wie primär chronische Polyarthritis, Arthritiden rheumatischen Ursprungs, Gelenkrheumatismus und Muskelrheumatismus mit ihren entsprechenden Schweregraden. Diese neuen Komplexe sind insbesondere wertvoll zur Schmerzentlastung, wie z. B. Kopfschmerzen, Dysmenorrhoe, postoperativer Schmerz, post-partum Schmerz und Schmerzen, die im Zusammenhang mit Grippe und Erkältungskrankheiten stehen.

Demgemäß beschreibt die Erfindung insbesondere einen anderen Aspekt zur Behandlung von Schmerz oder entzündlichem Fieber nach Verabreichung einer therapeutisch effektiven Dosis dieses Komplexes. Obwohl die genaue Dosis der pharmazeutisch aktiven Verbindung von einer Vielzahl von Parametern abhängig ist, z. B. Alter des Patienten, Zustand des Patienten, Anamnese und Compliance, liegt eine geeignete Dosis für sowohl orale als auch parenterale Verabreichungen von S-(+)-Ibuprofen-Komplex im Bereich von 50 - 1200 mg pro Tag, normalerweise zwischen 100 und 800 mg pro Tag, vorzugsweise zwischen 200 und 600 mg S-(+)-Ibuprofen pro Tag nach einmaliger oder mehrmaliger Verabreichung.

Bei einer topischen Anwendung dieses Komplexes liegt die entsprechende Dosis im Bereich von 10 - 200 mg pro Tag, im allgemeinen liegt die Dosis bei 20 - 100 mg pro Tag, je nach ärztlicher Anordnung.

Weitere Merkmale der Erfindung ergeben sich aus der folgenden Beschreibung anhand von Ausführungsbeispielen.

Beispiel 1:

Herstellung von Komplexen zwischen S-(+)-Ibuprofen und 1-Amino-1-desoxy-D-glucitol:

206,27 (250,0) mg S-(+)-Ibuprofen und 236,72 (181,19) mg 1-Amino-1-desoxy-D-glucitol werden in 6 ml Wasser gelöst und anschließend bei 45°C eine Stunde lange Ultraschall-behandelt. Die klare Lösung kann aufbewahrt und nach Sterilisation für medizinische Zwecke verwendet werden. Der Komplex kann aus der etherischen oder der alkoholischen Lösung kristallisiert werden durch Zugabe dieser Lösungsmittel bei 20°C unter ständigem Rühren zu einer wäßrigen Lösung aus S-(+)-Ibuprofen und 1-Amino-1-desoxy-D-glucitol (pH 7,5). Das mikrokristalline Präzipitat kann durch Filtration mit anschließender Trocknung über $CaCl_2$ unter $N_2$-Atmosphäre gesammelt werden. Falls keine kristallinen Formen erwünscht sind, kann das mikrokristalline Präzipitat zusätzlich zentrifugiert werden, der Überstand wird verworfen und das Präzipitat über $P_2O_5/CaCl_2$ bei 30°C getrocknet; der Schmelzpunkt des amorphen Komplexes ist 61°C, der der kristallinen Probe 59°C; bei Verwendung anderer Präzipitations-Lösungsmittel, z. B. Aceton oder Methyl-isopropyl-keton, DMF und Petrolether, wurden unterschiedliche kristalline Formen beobachtet, was einen bestimmten Grad an Polymorphismus dieser speziellen Komplexe offenbart, mit einem Schmelzpunkt von Fp: 106,5 - 107,5 °C. Die Verbindungen mit niedrigem Schmelzpunkt enthalten Hydratwasser in wechselnder molekularer Stöchiometrie.

Beispiel 2:

206,3 g (1 mol) S-(+)-Ibuprofen und 195,2 g (1 mol) D-(-)-N-methylglucamin werden mit 500 ml Isopropanol unter Rühren bis zum Sieden erhitzt, wobei eine klare Lösung entsteht. Unter Rühren werden 2,5 l n-Hexan zugefügt und das Gemisch erst 20 Minuten bei Raumtemperatur, dann 3 Stunden bei 0°C weitergerührt. Die ausgefallenen Kristalle werden abgesaugt, mit 2 x 150 ml n-Hexan gewaschen und bei Raumtemperatur getrocknet. Ausbeute: 398 - 400 g (99,1 - 99,6 % d. Th.), Schmp. 106,5 - 107,5°C.

Beispiel 3:

Herstellung einer Tablette:

| Zusammensetzung: | 1 Tablette enthält |
|---|---|
| wirksame Bestandteile | |
| S-(+)-Ibuprofen-N-Methylglukamin | 195 mg |
| = S-(+)-Ibuprofen | 100 mg |
| nicht wirksame Bestandteile | |
| Gelatine | 4 mg |
| Quervernetzte Natriumcarboxymethylcellulose | 17 mg |
| Magnesiumstearat | 4 mg |
| Gewicht pro Tablette | 220 mg |

Herstellung:

Die Gelatine wird zu 10 % in gereinigtem Wasser unter Erwärmen (max. 40°C) gelöst und dem Wirkstoff im Mischer bei geringer Mischleistung langsam zugefügt. Das erhaltene Granulat wird im Wirbelbett bei circa 40°C getrocknet und über eine Siebmaschine (Maschenweite 1,6 mm) abgesiebt. Das getrocknete Granulat wird zu Tabletten mit Hilfe von Stempeln (Durchmesser 8,4 mm) zu Tabletten mit 190 mg Endgewicht verpreßt.

Vorteilhaft können auch erfindungsgemäß die erfindungsgemäßen Komplexe in pharmazeutischen Zubereitungen verwendet werden, wie sie in der deutschen Anmeldung DE 40 15 794.6 beschrieben sind. Solche isotropen Lösungen können hergestellt werden durch die folgenden Verfahrensschritte:

a.) Erwärmen des Trägers unter Rühren bis oberhalb des Schmelzpunktes, bis eine isotrope, transparente Flüssigkeit vorliegt;
b.) Messung der elektrischen Leitfähigkeit und der Viskosität bei der Temperatur des Schmelzpunktes, um das Vorliegen einer isotropen, transparenten Flüssigkeit zu gewährleisten;
c.) Bestimmung des refraktiven Index;
d.) Einstellen der gewünschten Konzentration des pharmazeutischen Wirkstoffs unter Beachtung des Molenbruches, der bei 37° C zwischen 0,001 und 0,67 liegen muß ;
e.) Eintragen des pharmazeutischen Wirkstoffes unter stetigem Rühren in das Lösungsmittel;
f.) Rühren des Ansatzes, bis der pharmazeutische Wirkstoff gelöst und eine transparente Lösung entstanden ist;
g.) Messung des differentiellen refraktiven Index-Inrementes $[(\Delta n/\Delta c)_{T/P=konstant}]$ zur Bestimmung der monomolekularen Lösung und/oder
h.) Kontrolle der nativen Konformation und der Monomolekularität des pharmazeutischen Wirkstoffes in der Lösung durch Messung des molaren Extinktionskoeffizienten im UV-Bereich und durch Aufnahme des Absorptionsspektrums sowie Feststellen der chiralen Konfiguration durch Messung im Polarimeter und/oder
i.) Messung der Trübung, um eine homogene Lösung sicherzustellen und/oder
k.) Messung der spezifischen Leitfähigkeit $[(\Omega)_{T,V=konstant}]$ zur Kontrolle der ionalen Konzentration in der isotropen Lösung;
l.) Abkühlen der klaren Lösung und Herstellung einer galenischen Formulierung ;
m.) weiteres Abkühlen der Lösung auf Raumtemperatur, bis die Lösung erstarrt ist.

In der Offenlegungsschrift 21 03 387 vom 17. August 1972 werden pharmazeutische Zubereitungen zur Behandlung von degenerativen Gelenkerkrankungen in Kombination eines oder mehrerer nichtsteroidaler Antirheumatika, z.B. Diphenylbutazon, Monophenylbutazon, Indometacin etc. mit Glukosamin-Hydrochlorid im Molverhältnis von 1:10 bis zu 10:1 offenbart. Im Gegensatz zu dieser Herstellungsvorschrift wird entsprechend der hier vorliegenden, neuen Erfindung ein Komplex zwischen z.B. S-(+)-Ibuprofen und $\alpha$-D-N-methylglucosamin oder $\alpha$-D-Aminozuckern im Molverhältnis von S-(+)-Ibuprofen zu $\alpha$-D-Aminozucker von 1:1 hergestellt. Dieser Komplex ist z.B entsprechend eines offenbarten Beispiels (siehe Beispiel 2) hergestellt und anschließend kristallisiert worden. Die folgende Röngtenstrukturanalyse ergab eine chirale Raumgruppe mit den Zell-Dimensionen a = 8.275Å, b =40.872Å und c = 6.520Å, Raumgruppe $P2_12_12_1$ (#19), mit vier Komplex-Molekülen, bestehend aus S-(+)-Ibuprofen und -D-N-Methyl-Glukamin (oder $\alpha$-D-Glukosamin)

im Verhältnis 1:1, in der Einheitszelle (Fig. 2). Entsprechende Ergebnisse sind auch für R-(-)-Ibuprofen und α-D-Glukamin (oder α-D-Glukamin (oder α-D-Galaktosamin) erhalten worden (Fig. 1). Diese Strukturen zeigen, daß es sich um einen Wasserstoffbrücken-gebundenen Komplex handelt, und zwar teilen sich die Karboxylgruppe des S oder R-Ibuprofens ein Proton mit der Hydroxylgruppe am Kohlenstoffatom ($C_3$) des Zuckers, so daß wir hier ein "Proton-Switch" der Form

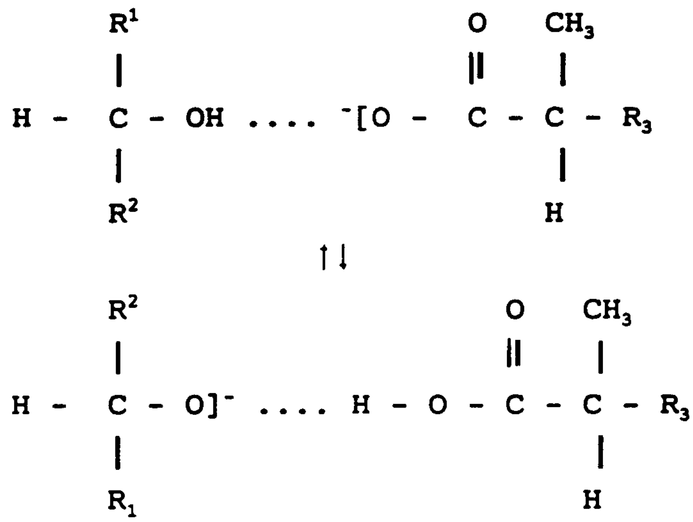

zu beobachten ist, welcher den ganzen Molekülkomplex neutral erscheinen läßt. Hiermit ist eindeutig gezeigt, daß es sich weder um Ionenpaare, noch um Salze, sondern um einen 1:1 Komplex mit einer starken Wasserstoffbrückenbildung zwischen der Karboxo-Gruppe des S- oder R-Ibuprofens unddem $O_3$-Sauerstoff des N-Methyl-Amino-S-desoxy-D-Glucitols (N-Methyl-D-Glukamin) handelt. Wie aus der Struktur ersichtlich, ist die Amino-Gruppe bei dieser Komplexbildung nicht beteiligt. Dieser überraschende Befund steht auch im Einklang mit FT-IR-Untersuchungen als auch mit Raman-sprektroskopischen Experimenten. Darüber hinaus ist es erstaunlich, daß dieser Molekülkomplex, auch mit nicht substitierten 2-Amino-2-desoxy-Glukose oder dem Stereoisomer 2-Amino-2-desoxy-galaktose, den Zuckeranteil in der offenkettigen Form zeigt und nicht in seiner Zyklischen Konformation, was bislang nicht bekannt war. Die pharmakokinetische und pharmakodynamische Verhaltensweise ist die der Komplexe bestehend aus α-D-Aminosäuren und S-(+)-Ibuprofen sehr ähnlich: rascher Wirkungseintritt mit einem $t_{max}$ von 15- 20 Minuten, eine hohe AUC von 55.= µg/ml x h bei gleicher Wirkstoffmenge (150 mg). Die Tabelle 1 zeigt alle pharmakokinetischen Daten, welche relevant sind und

die Überlegenheit des Zuckerkomplexes gegenüber der freien Säure demonstrieren.

## Tabelle 1

**Pharmakokinetische Parameter nach Einnahme einer (single) oralen Dosis von S-(+)-Ibuprofen-N-methyl-2-desoxy-glucitol (150 mg Ibuprofen Wirkstoff) (4 Probanden)**

**Tablette S-(+)-Ibuprofen freie Säure**

| Mean ± SD | Tablette, freie Säure | Zucker-Komplex |
|---|---|---|
| $t_{max}$, h | 2,1 ± 0,2 | 0,25 ± 0,11 |
| $C_{max}$, µg/ml | 10,1 ± 5,0 | 24,5 ± 6,7 |
| AUC, µg/ml x h | 40,0 ± 11,0 | 55,0 ± 10,2 |
| $t_{lag}$, h | 0,50 ± 0,1 | 0,1 ± 0,02 |
| $t_{1/2}$, h | 2,2 ± 0,3 | 1,5 ± 0,3 |

## Patentansprüche

1. Wasserstoffbrücken-gebundene Komplexe mit einer Stöchiometrie von 1:1 aus S-(+)-Phenyl-alkansäuren und Aminozuckern, bei welchen die Komplex-Bindung auf Wechselwirkungen zwischen der Karboxylgruppe der S-(+)-Phenyl-alkansäuren und der Hydroxylgruppe am Kohlenstoffatom ($C_3$) der Aminozucker mit einem Proton-Switch der Form

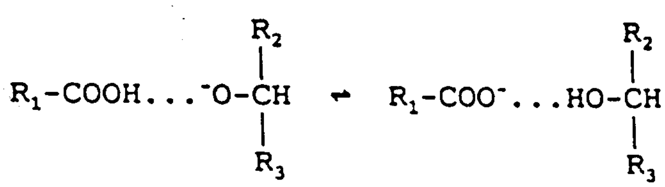

beruht, wobei $R_1$-COOH die S-(+)-Phenyl-alkansäuren und

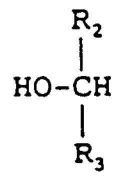

die Aminozucker bedeuten, und wobei die pKa-Werte bezüglich der Karboxylgruppe der S-(+)-Phenyl-alkansäuren im Bereich von 3,5 - 3,9 und die pKa-Werte bezüglich der Hydroxylgruppe am Kohlenstoffatom ($C_3$) der Aminozucker im Bereich von 1,9 - 4,0 liegen.

**2.** Komplexe nach Anspruch 1,
dadurch gekennzeichnet,
daß als S-(+)-Phenyl-alkansäuren S-(+)-Ibuprofen oder S-(+)-Naproxen verwendet werden.

**3.** Komplexe nach Anspruch 1 oder 2,
dadurch gekennzeichnet,
daß die Aminozucker die folgende allgemeine Formel aufweisen

Z-NHR,

wobei

R = Wasserstoff, Methyl oder Ethyl und
Z = das Gerüst des Aminozuckers, der 5 oder 6 Kohlenstoffatome enthält.

**4.** Komplexe nach einem oder mehreren der vorhergehenden Ansprüche,
dadurch gekennzeichnet,
daß der Aminozucker linear oder zyklisch ist oder in der D-Form vorliegt.

**5.** Komplexe nach einem oder mehreren der vorhergehenden Ansprüche,
dadurch gekennzeichnet,
daß der Aminozucker ein Pentose- oder Hexosederivat, oder ein Epimer der Hexosamine, oder der Pentosamine ist.

**6.** Komplexe nach einem oder mehreren der vorhergehenden Ansprüche,
dadurch gekennzeichnet,
daß der Aminozucker ausgewählt wird aus Glukamin, N-Methyl-Glukamin, N-Ethyl-Glukamin, Ribamin, D-Ribamin, Allosamin, Altrosamin, Glukosamin, Mannosamin, Gulosamin, Idosamin, Galaktosamin, Talosamin, Ribosamin, Arabinosamin, Xylosamin und Lyxosamin.

**7.** Komplexe nach einem oder mehreren der vorhergehenden Ansprüche,
dadurch gekennzeichnet,
daß die S-(+)-Phenyl-alkansäuren von der Form

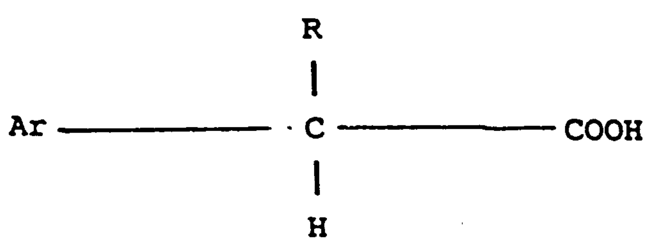

sind, in welcher R ein Alkylrest ist, Ar eine monozyklische, polyzyklische oder ortho-kondensierte polyzyklische aromatische Gruppe mit bis zu 12 Kohlenstoffatomen in dem aromatischen System, wie Phenyl, Diphenyl und Naphtyl ist und wobei die Substituenten dieser aromatischen Gruppen ein oder mehrere Halogenatome, $C_1$-$C_4$-Alkyle, Benzyl, Hydroxy, $C_1$-$C_2$-Alkoxy, Phenoxy und Benzoyl-Gruppen enthalten.

**8.** Komplexe nach einem oder mehreren der vorangegangenen Patentansprüche,
dadurch gekennzeichnet,
daß die substituierten Aryle 4-Isobutyl-phenyl, 3-Phenoxy-phenyl, 2-Fluoro-4-diphenyl, 4'-Fluoro-4-diphenyl, 6-Methoxy-2-naphthyl, 5-Chloro-6-methoxy-2-naphthyl und 5-Bromo-6-methoxy-naphthyl, 4-Chloro-phenyl, 4-Difluoro-methoxy-phenyl, 6-Hydroxy-2-naphthyl und 5-Bromo-6-hydroxy-2-naphthyl sind.

**9.** Verfahren zur Herstellung der Komplexe nach einem oder mehreren der Ansprüche
gekennzeichnet durch
folgende Verfahrensschritte:

a) zur Herstellung aus wäßrigem Medium (nur Wasser) oder schwach gepufferten wäßrigen Lösungen, die einen pH-Bereich umfassen zwischen pH 5.5-7.5 (20°C) bereitet man eine gepufferte, wäßrige Lösung vor, in die unter stetem Rühren eine äquivalente Menge S-(+)-Phenyl-alkansäure eingetragen wird;
b) die Lösung wird unter stetem Rühren auf 40°C (Wasserbad) erwärmt, bis eine klare, transparente Lösung erhalten wird und alle S-(+)-Phenyl-alkansäure in Lösung gegangen ist;
c) anschließend wird der pH der Lösung auf pH 5.5-6.O unter Zugabe von verdünnter Phosphorsäure ($H_3PO_4$) eingestellt (20°C) und danach wird die äquivalente (entsprechende) Menge der Aminozucker unter stetem Rühren eingetragen;
d) die Komplexbildung ist nach 20 Minuten beendet, worauf nach Abkühlen auf 0-4°C die Komplexe kristallin anfallen und über eine Glasnutsche oder einen Glasfilter (1G4) von der Mutterlauge abgetrennt werden können;
e) alternativ zu Verfahrensschritt d) kann man die klare Lösung im Rotationsverdampfer (Wasserbad-Temperatur 25 - 30°C) im Wasserstrahl-Vakuum auf die Hälfte des Volumens eingeengt werden, worauf sich ein farbloser (amorpher) Niederschlag bildet, der über einen 1G4-Glasfilter abfiltriert aus Wasser/Ethanol ($^{70}/_{30}$ $^{V}/_{V}$) oder aus Ethylacetat (100 %) umkristallisiert werden kann.

**10.** Pharmazeutische Zubereitung enthaltend einen oder mehrere Komplexe nach einem oder mehreren der vorhergehenden Ansprüche und gegebenenfalls wahlweise zusätzlich physiologisch verträgliche gebräuchliche Streckungsmittel oder Träger.

**11.** Pharmazeutische Zubereitung nach Anspruch 10 auf der Grundlage von Phenyl-alkansäuren mit antiinflammatorischer, antipyretischer, antimikrobieller und analgetischer Wirkung, enthaltend einen Wirkstoffkomplex aus einer Phenyl-alkansäure und einem Aminozucker und ggf. zusätzlich übliche und physiologisch verträgliche Hilfsstoffe,
dadurch gekennzeichnet,
daß der Wirkstoffkomplex aus S-(+)-Phenyl-alkansäuren und Aminozuckern besteht.

**12.** Pharmazeutische Zubereitung nach Anspruch 10 oder 11 auf der Grundlage von Ibuprofen oder Naproxen mit antiinflammatorischer, antipyretischer, antimikrobieller und analgetischer Wirkung, enthaltend einen Wirkstoffkomplex aus einer Ibuprofen oder Naproxen und Aminozuckern und ggf. zusätzlich übliche und physiologisch verträgliche Hilfsstoffe,
dadurch gekennzeichnet,
daß der Wirkstoffkomplex aus S-(+)-Ibuprofen oder S-(+)-Naproxen und einem Aminozucker besteht und einen Gewichtsanteil 0,1 bis 90% (w/w) der Zusammensetzung ausmacht.

**13.** Pharmazeutische Zusammensetzung nach einem oder mehreren der Ansprüche 10 bis 12,
dadurch gekennzeichnet,
daß sie 50 bis 800 mg, S-(+)-Ibuprofen oder S-(+)-Naproxen enthält.

**14.** Pharmazeutische Zubereitung nach einem oder mehreren der Ansprüche 10, bis 13,
dadurch gekennzeichnet,
daß die geeignete Dosis für die orale oder die parenterale Verabreichung im Bereich von 50 - 1200 mg pro Tag, S-(+)-Ibuprofen pro Tag liegt und daß die geeignete Dosis bei einer topischen Anwendung des Komplexes im Bereich von 10 - 200 mg pro Tag liegt.

**15.** Pharmazeutische Zubereitung nach einem oder mehreren der Ansprüche 10 - 14
bestehend aus einem oder mehreren Komplexen als Wirkstoffen und einem Träger, der im Bereich zwischen 20

und 80°C erstarrt und wasserlöslich ist, dadurch gekennzeichnet,
daß sie eine isotrope Lösung ist, wobei

a) der Wirkstoff in dem Träger monomolekular oder als Ion gelöst ist,
b) der Wirkstoff in seiner nativen Konformation und/oder seiner biologisch aktiven chiralen (enantiomeren) Konformation vorliegt,
c) der Wirkstoff einen Molenbrucn von 0,001 bis 0,67 bei 37°C aufweist,
d) der Träger bei Körpertemperatur schmelzflüssig, phaseneinheitlich und isotrop ist,
e) die isotrope Lösung, bestehend aus Träger und Wirkstoff, bei Raumtemperatur erstarrt,
f) die erstarrte Lösung kristallin oder nicht-kristallin ist oder sie den Wirkstoff kristallin enthält oder sie den Wirkstoff auskristallisieren kann,
g) die monomolekulare oder ionische Lösung einen osmotischen Druck hat und eine molare Gefrierpunkterniedrigung bewirkt,
h) der gelöste Wirkstoff innerhalb des Polymerelektrolyten einen temperaturabhängigen Diffusionskoeffizienten hat und eine temperaturabhängige spezifische Leitfähigkeit hat.

## Claims

1. Hydrogen-bridge-bound complexes having a stoichiometry of 1:1 comprising S(+)-phenyl alkane acids and amino sugars in which the complex bond is based on interactions of the carboxyl group of the S(+)-phenyl alkane acids and the hydroxyl group at the carbon atom ($C_3$) of the amino sugars having a proton switch of the form

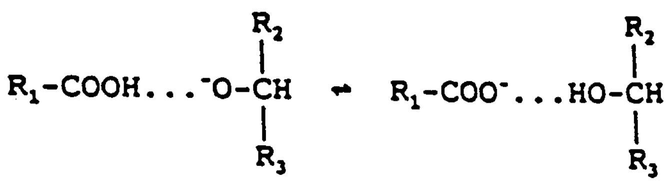

where $R_1$-COOH denotes the S(+)-phenyl alkane acids and

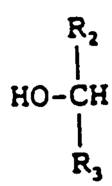

denotes the amino sugars, the pKa values relating to the carboxyl group of the S(+)-phenyl alkane acids lying in the range of 3.5 - 3.9 and the pKa values relating to the hydroxyl group at the carbon atom ($C_3$) of the amino sugars lying in the range of 1.9 - 4.0.

2. Complexes according to claim 1,
characterized in
that as S(+)-phenyl alkane acids S(+)-ibuprofen or S(+)-naproxen are used.

3. Complexes according to claim 1 or 2,
characterized in
that the amino sugars have the following general formula:

Z-NHR

where

R = hydrogen, methyl or ethyl and
Z = the skeleton of the amino sugar, which contains 5 or 6 carbon atoms.

4. Complexes according to one or more of the preceding claims, characterized in that the amino sugar is linear or cyclic or in the D-form.

5. Complexes according to one or more of the preceding claims, characterized in that the amino sugar is a pentose or hexose derivative or an epimer of the hexosamines or the pentosamines.

6. Complexes according to one or more of the preceding claims, characterized in that the amino sugar is selected from glucamine, N-methyl glucamine, N-ethyl-glucamine, ribamine, D-ribamine, allosamine, altrosamine, glucosamine, mannosamine, gulosamine, idosamine, galactosamine, talosamine, ribosamine, arabinosamine, xylosamine and lyxosamine.

7. Complexes according to one or more of the preceding claims, characterized in that the S(+)-phenyl alkane acids have a structure of the form

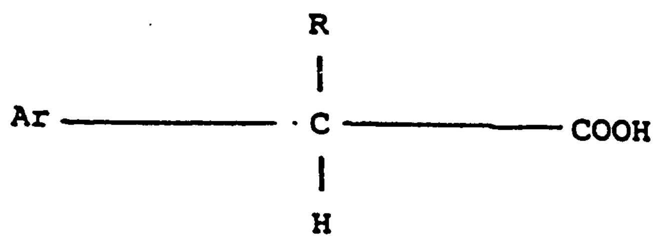

in which R is an alkyl residue, Ar is a monocyclic, polycyclic or ortho-condensed polycyclic aromatic group having up to twelve carbon atoms in the aromatic system, like phenyl, diphenyl, and naphthyl, and whereby the substituents of these aromatic groups comprise one or more halogen atoms, $C_1$-$C_4$-alkyls, benzyl, hydroxy, $C_1$-$C_2$ alkoxy, phenoxy and benzoyl groups.

8. Complexes according to one or more of the preceding claims, characterized in that the substituted aryls are: 4-isobutyl-phenyl, 3-phenoxy-phenyl, 2-fluoro-4-diphenyl, 4'-fluoro-4-diphenyl, 6-methoxy-2-naphthyl, 5-chloro-6-methoxy-2-naphthyl and 5-bromo-6-methoxy-naphthyl, 4-chloro-phenyl, 4-difluoromethoxy-phenyl, 6-hydroxy-2-naphthyl, and 5-bromo-6-hydroxy-2-naphthyl.

9. Method for the preparation of the complexes according to one or more of the preceding claims, characterized by the following method steps:

   a) for the preparation from aqueous medium (only water) or weakly buffered aqueous solutions covering a pH range between pH 5.5 - 7.5 (20°C) a buffered aqueous solution is prepared and into it an equivalent amount S(+)-phenyl alkane acid is introduced with constant stirring;
   b) the solution is heated with constant stirring to 40°C (water bath) until a clear transparent solution is obtained and all the S(+)-phenyl alkane acid has gone into solution;
   c) thereafter the pH of the solution is adjusted to pH 5.5 - 6.0 by addition of diluted phosphoric acid ($H_3PO_4$) (20°C) and then the equivalent (corresponding) amount of the amino sugar is introduced with constant stirring;
   d) the complex formation is terminated after 20 minutes, whereupon after cooling to 0 - 4°C the complexes precipitate in crystalline form and can be separated from the mother liquor via a sintered glass funnel or glass filter (1G4);
   e) alternatively to method step d) the clear solution can be reduced in a rotary evaporator (water bath temperature 25 - 30°C) in the water jet vacuum to half the volume, whereupon a colourless (amorphous) deposit forms which is filtered off via a 1G4 glass filter and can be recrystallized from water/ethanol (70/30 v/v) or from ethyl acetate (100%).

10. Pharmaceutical preparation containing one or more complexes according to one or more of the preceding claims and possibly optionally additionally physiologically compatible extenders or carriers.

**11.** Pharmaceutical preparation according to claim 10 on the basis of phenyl alkane acids having anti-inflammatory, antipyretic, antimicrobial and analgesic effect, containing an active substance complex comprising a phenyl alkane acid and an amino sugar and possibly additionally usual physiologically compatible auxiliary substances, characterized in that the active substance complex consists of S(+)-phenyl alkane acids and amino sugars.

**12.** Pharmaceutical preparation according to claim 10 or 11 on the basis of ibuprofen or naproxen with anti-inflammatory, antipyretic, antimicrobial and analgesic effect, containing an active substance complex comprising an ibuprofen or naproxen and amino sugars and possibly additionally usual physiologically compatible auxiliary substances, characterized in that the active substance complex consists of S(+)-ibuprofen or S(+)-naproxen and an amino sugar and represents an amount by weight of 0.1 to 90 % (w/w) of the composition.

**13.** Pharmaceutical preparation according to one or more of claims 10 to 12, characterized in that it contains 50 to 800 mg S(+)-ibuprofen or S(+)-naproxen.

**14.** Pharmaceutical preparation according to one or more of claims 10 to 13, characterized in that the suitable dose for oral or parenteral administration is in the range of 50 - 1200 mg daily, S(+)-ibuprofen daily and that the suitable doses for a topical administration of the complex lies in the range of 10 - 200 mg daily.

**15.** Pharmaceutical preparation according to one or more of claims 10 to 14, consisting of one or more complexes as active substances and a carrier which solidifies in the range between 20 and 80°C and is water-soluble, characterized in that it is an isotropic solution, wherein

a) the active substance is dissolved in the carrier in monomolecular form or as ion,
b) the active substance is present in its native conformation and/or its biologically active chiral (enantiomeric) conformation,
c) the active substance has a molar fraction of 0.001 to 0.67 at 37°C,
d) the carrier is molten, phase-uniform and isotropic at body temperature,
e) the isotropic solution, consisting of carrier and active substance, solidifies at room temperature,
f) the solidified solution is crystalline or non-crystalline and contains the active substance in crystalline form or can crystallize the active substance out,
g) the monomolecular or ionic solution has an osmotic pressure and effects a molar freezing point reduction,
h) the dissolved active substance within the polymer electrolyte has a temperature-dependent diffusion coefficient and a temperature-dependent specific conductivity.

## Revendications

**1.** Complexes d'acides S-(+)-phénylalcanoïques et de sucres aminés liés par des liaisons hydrogènes avec une stoechiométrie de 1:1, dans lesquels la formation du complexe est basée sur les interactions entre le groupe carboxyle des acides S-(+)-phénylalcanoïques et le groupe hydroxyle sur l'atome de carbone ($C_3$) du sucre aminé avec un échange de proton de la forme

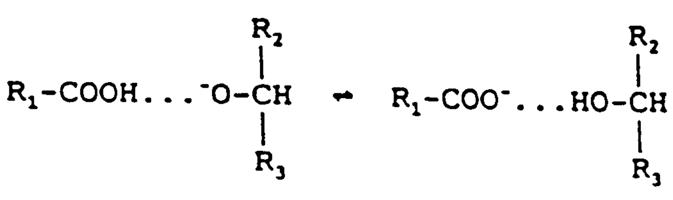

dans lesquels $R_1$-COOH représente les acides S-(+)-phénylalcanoïques et

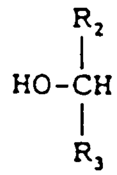

représente le sucre aminé et dans lesquels le pKa des groupes carboxyles des acides S-(+)-phénylalcanoïques sont compris entre 3,5 et 3,9 et les pKa du groupe hydroxyle sur l'atome de carbone ($C_3$) du sucre aminé sont compris entre 1,9 et 4,0.

**2.** Complexes selon la revendication 1,
caractérisés en ce que,
le S-(+)-ibuprofène ou le S-(+)-naproxène sont utilisés à la place des acides S-(+)-phénylalcanoïques.

**3.** Complexe selon la revendication 1 ou 2, caractérisé en ce que,
les sucres aminés ont la formule générale suivante

Z-NHR,

dans laquelle
R = hydrogène, méthyle ou éthyle et
Z = la structure du sucre aminé contenant 5 ou 6 atomes de carbone.

**4.** Complexes selon une ou plusieurs des revendications précédentes,
caractérisés en ce que,
le sucre aminé est linéaire ou cyclique ou de la forme D.

**5.** Complexes selon une plusieurs des revendications précédentes,
caractérisés en ce que,
le sucre aminé est un pentose ou un dérivé d'hexose ou un épimère de l'hexosamine ou de la pentosamine.

**6.** Complexes selon une plusieurs des revendications précédentes,
caractérisés en ce que,
le sucre aminé est choisi dans l'ensemble constitué par la glucamine, la N-méthyl-glucamine, la N-éthyl-glucamine, la ribamine, la D-ribamine, l'allosamine, l'altrosamine, la glucosamine, la mannosamine, la gulosamine, l'idosamine, la galactosamine, la talosamine, la ribosamine, l'arabinosamine, la xylosamine et la lyxosamine.

**7.** Complexes selon une ou plusieurs des revendications précédentes,
caractérisés en ce que,
les acides S-(+)-phénylalcanoïques sont de la forme

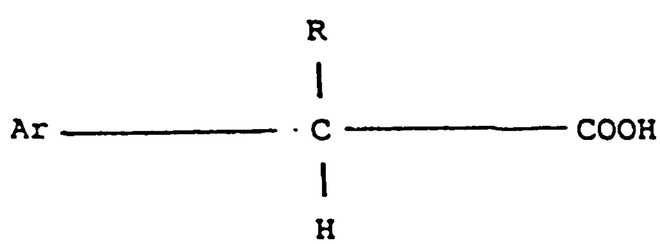

dans laquelle R est un résidu alkyle, Ar est un groupe aromatique monocyclique, polycyclique ou polycyclique orthocondensé ayant jusqu'à 12 atomes de carbone dans le système aromatique, tel que le phényle, le diphényle et le naphtyle et dans lesquels les substituants de ces groupes aromatiques renferment un ou plusieurs atomes d'halogènes, des groupes $C_1$-$C_4$-alkyle, benzyle, hydroxy, $C_1$-$C_2$-alcoxy, phénoxy et benzoyl.

8. Complexes selon une ou plusieurs des revendications précédentes, caractérisés en ce que, les aryles substitués sont le 4-isobutyl-phényle, le 3-phénoxy-phényl, le 2-fluoro-4-diphényle, le 4'-fluoro-4-diphényle, le 6-méthoxy-2-naphtyle, le 5-chloro-6-méthoxy-2-naphtyle et le 5-bromo-6-méthoxy-naphtyle, le 4-chloro-phényle, le 4-difluoro-méthoxy-phényle, le 6-hydroxy-2-naphtyle et le 5-bromo-6-hydroxy-2-naphtyle.

9. Procédé de fabrication de complexes selon une plusieurs des revendications caractérisé par les étapes de fabrication suivantes:

   a) pour la fabrication en milieu aqueux (seulement de l'eau) ou en solutions aqueuses faiblement tamponnées dont le pH est compris entre 5,5 et 7,5 (20°C), on prépare une solution aqueuse tamponnée dans laquelle on rajoute une quantité équivalente d'acide S-(+)-phénylalcanoïque avec une agitation constante;
   b) la solution est chauffée à 40°C (au bain-marie) avec une agitation constante jusqu'à l'obtention d'une solution claire, transparente et que tout l'acide S-(+)-phénylalcanoïque soit mis en solution;
   c) ensuite le pH de la solution est ajusté à 5,5-6,0 par addition d'acide phosphorique dilué ($H_3PO_4$) (20°C) puis une quantité équivalente (correspondante) d'acide $\alpha$-hydroxyalcanoïque est rajoutée avec une agitation constante.
   d) la formation du complexe est réalisée au bout de 20 minutes après quoi le complexe est précipité en cristaux par refroidissement à 0-4°C et peut être séparé de la solution mère sur un entonnoir filtre en verre ou sur un filtre en verre (1G4);
   e) alternativement à l'étape de fabrication d) la solution claire peut être réduite à la moitié de son volume dans un évaporateur rotatif (température du bain-marie 25 - 30°C) sous une trompe à vide, ce qui fait apparaître un précipité incolore (amorphe) qui est filtré sur un filtre en verre 1G4 et peut être cristallisé dans un mélange eau/éthanol (70/30 V/V) ou dans de l'acétate d'éthyle (100%).

10. Composition pharmaceutique renfermant un ou plusieurs complexes selon une ou plusieurs des revendications précédentes et, au choix, un diluant ou un porteur courant physiologiquement compatibles.

11. Composition pharmaceutique selon la revendication 10 à base d'acides phényl-alcanoïques ayant une action anti-inflammatoire, antipyrétique, antimicrobienne et analgésique, comprenant un complexe actif d'un acide phényl-alcanoïque et d'un sucre aminé et, le cas échéant, un agent auxiliaire supplémentaire, courant et physiologiquement compatible, caractérisé en ce que, le complexe actif se compose d'acides S-(+)-phénylalcanoïque et de sucres aminés.

12. Composition pharmaceutique selon la revendication 10 ou 11 à base d'ibuprofène ou de naproxène ayant une action anti-inflammatoire, antipyrétique, antimicrobienne et analgésique, comprenant un complexe actif d'ibuprofène ou de naproxène et de sucres aminés et, le cas échéant, un agent auxiliaire supplémentaire, courant et physiologiquement compatible, caractérisée en ce que, le complexe actif se compose de S-(+)-ibuprofène ou S-(+)-naproxène et d'un sucre aminé et représente 0,1 à 90% en poids (p/p) de la préparation.

13. Composition pharmaceutique selon une ou plusieurs des revendications 10 à 12, caractérisée en ce que, elle renferme 50 à 800 mg de S-(+)-ibuprofène ou de S-(+)-naproxène.

14. Composition pharmaceutique selon une ou plusieurs des revendications 10 à 13, caractérisée en ce que, la dose adaptée à l'administration orale ou parentérale est de l'ordre de 50 à 1200 mg par jour de S-(+)-ibuprofène et la dose adaptée à l'utilisation topique du complexe est de l'ordre de 10 à 200 mg par jour.

15. Composition pharmaceutique selon une ou plusieurs des revendications 10 à 14, composée d'un ou plusieurs complexes servant d'agents actifs et d'un porteur qui fige dans le domaine de température compris entre 20 et 80°C et qui est soluble dans l'eau, caractérisé en ce que, c'est une solution isotrope dans laquelle,

a) l'agent actif est dissout sous forme d'ion ou sous forme monomoléculaire,

b) l'agent actif se présente sous sa conformation native et/ou sous sa conformation chirale biologiquement active (énantiomère),

c) l'agent actif présente une fraction de mole de 0,001 à 0,67 à 37°C,

d) l'agent porteur est liquéfié, en une seule phase et isotrope à la température corporelle,

e) la solution isotrope, composée d'un porteur et d'un agent actif se fige à la température ambiante,

f) la solution figée est cristalline ou non cristalline ou elle renferme l'agent actif sous forme cristalline ou elle peut cristalliser l'agent actif,

g) la solution monomoléculaire ou ionique a une tension osmotique et diminue le point de congélation molaire,

h) l'agent actif dissout dans le polymère électrolytique a un coefficient de diffusion dépendant de la température et une conductibilité spécifique dépendante de la température.

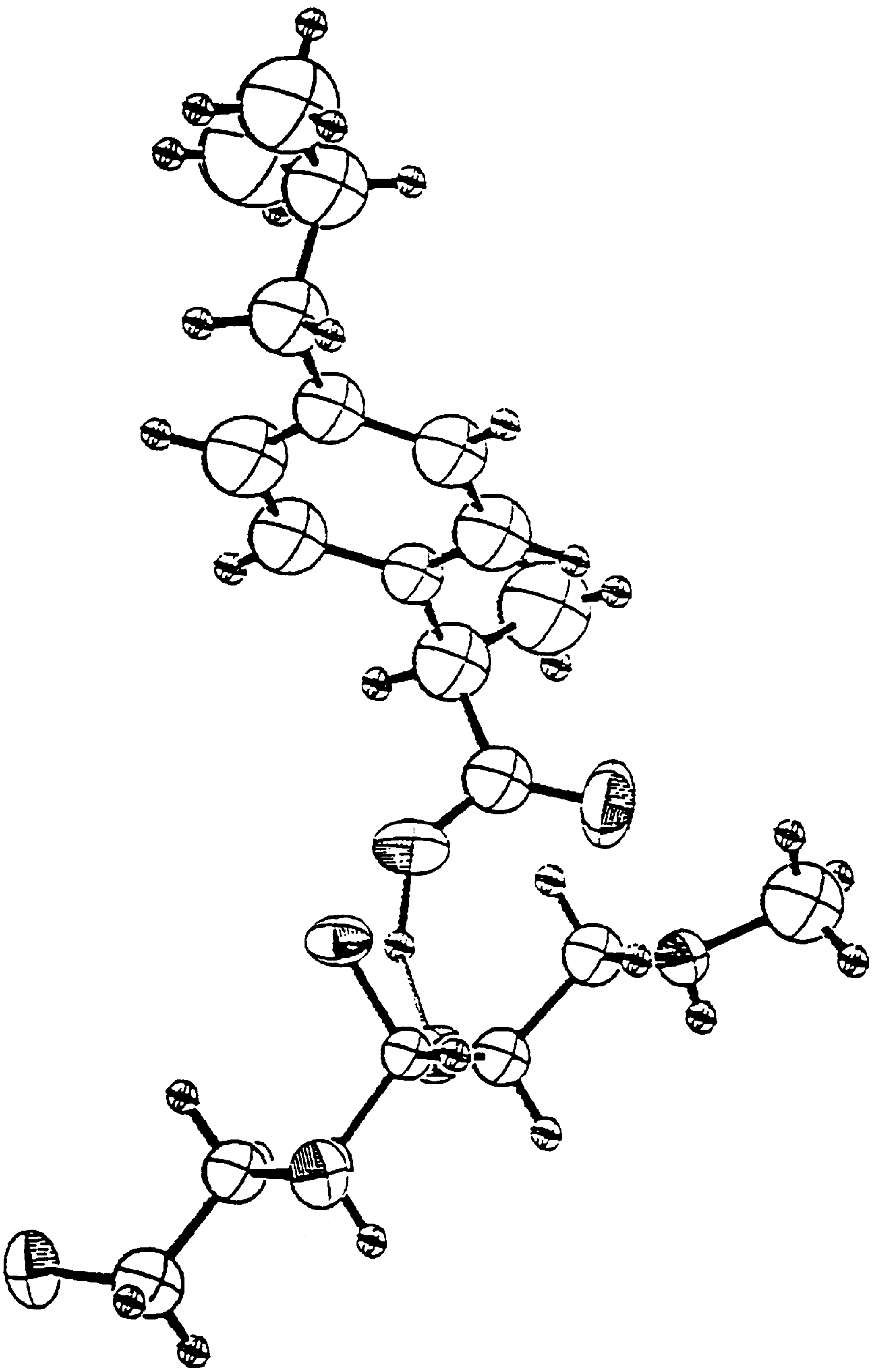

Fig 1

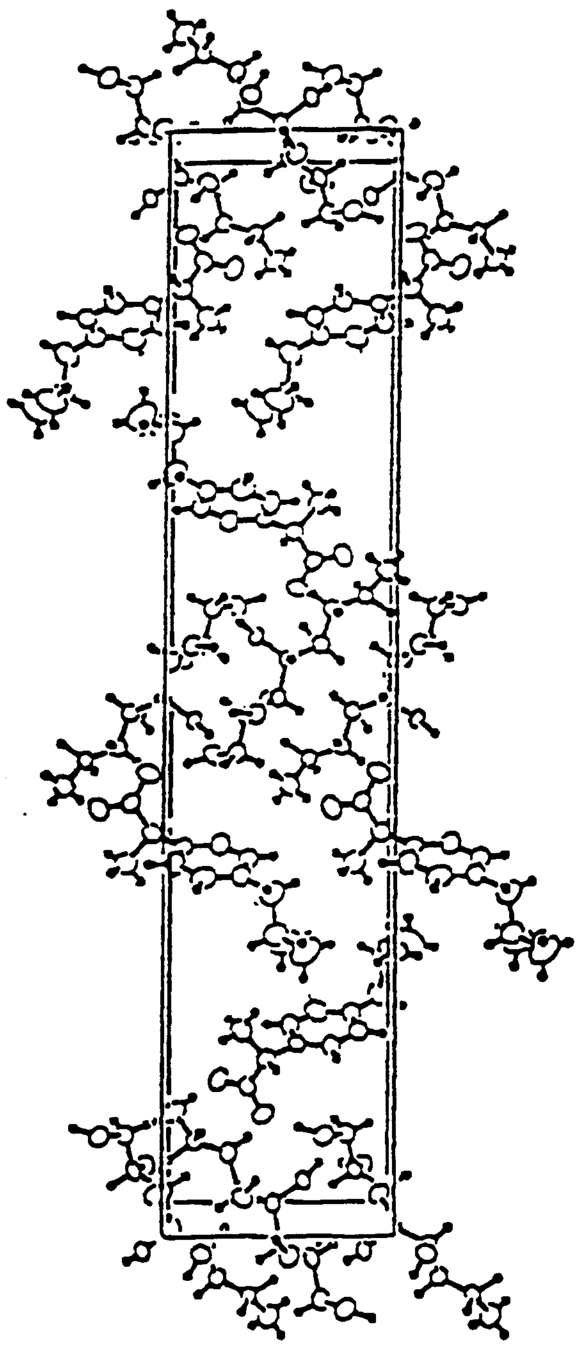

Fig 2